# EUROPEAN PATENT SPECIFICATION

(11) **EP 3 115 385 B1**
(45) Date of publication and mention of the grant of the patent: **25.05.2022**
(21) Application number: 15758164.6
(22) Date of filing: 03.03.2015
(51) Int. Cl.: C09J 175/04, C08G 18/02, C08G 18/76, C08G 18/38, C08G 18/30, C08G 18/18, C08G 18/12, C08G 18/09, C07D 251/32, B32B 7/12, B32B 27/32, B32B 15/20, B32B 15/085, B32B 37/12, C08G 18/70

(54) **POLYISOCYANURATE COMPOSITION AND METHOD FOR PRODUCING SAME**
POLYISOCYANURATZUSAMMENSETZUNG UND VERFAHREN ZUR HERSTELLUNG DAVON
COMPOSITION DE POLYISOCYNURATE ET SON PROCÉDÉ DE PRODUCTION

(30) Priority: 04.03.2014 JP 2014041952; 04.03.2014 JP 2014041957
(43) Date of publication of application: 11.01.2017
(73) Proprietor: Mitsui Chemicals, Inc., Minato-ku Tokyo 105-7122 (JP)
(72) Inventor: YOSHIDA, Tsutomu, Sodegaura-shi Chiba 299-0265 (JP); NAKASHIMA, Tatsuya, Sodegaura-shi Chiba 299-0265 (JP); MORIYA, Toshiaki, Sodegaura-shi Chiba 299-0265 (JP)
(74) Representative: Hoffmann Eitle
(86) International application number: PCT/JP2015/056252
(87) International publication number: WO 2015/133493

(56) References cited:
- WO-A1-2013/024108
- WO-A2-2013/079614
- WO-A2-2013/079614
- JP-A- H1 087 782
- JP-A- H06 299 116
- JP-A- H09 227 801
- JP-A- H10 176 027
- JP-A- H11 171 966
- JP-A- S61 129 173
- JP-A- S61 129 173
- JP-A- 2012 073 567
- JP-A- 2014 024 930
- JP-A- 2014 205 854

## Description

### TECHNICAL FIELD

The present invention relates to a method for producing a polyisocyanurate composition and a polyisocyanurate composition produced by the method for producing a polyisocyanurate composition.

### BACKGROUND ART

Polyurethane resin is generally produced by reaction of polyisocyanate and an active hydrogen group-containing compound, and is widely used in various industrial fields as, for example, coatings, adhesives, and elastomers.

For polyisocyanate used in production of polyurethane resin, for example, xylylenediisocyanate is known, and it has been proposed that xylylenediisocyanate is modified into an isocyanurate product to be used.

To be more specific, Patent Document 1 (see below) has proposed, for example, a method for producing polyisocyanate containing an isocyanurate group by allowing m-xylylenediisocyanate to react in the presence of tetrabutylphosphoniumdifluoride at 70°C or less, and thereafter adding dibutylphospate to further allow reaction at 70°C; and a method for producing polyurethane by allowing the polyisocyanate to react with polyol.

### Citation List

### Patent Document

Patent Document 1: WO2012/010524

WO 2013/079614 is directed to a procedure for the continuous preparation of oligomeric or polymeric di- and/or triisocyanates by catalytic modification of monomeric di- and/or triisocyanates. The procedure is useful for aliphatic or aromatic di-/triisocyanates, and employs quaternary phosphonium or ammonium compounds as catalysts.

JP-S61-129173 relates to the preparation of an isocyanurate compound by reacting an isocyanate compound in the presence of a hydroxyalkyl quaternary ammonium compound and an organic phosphite.

JP HI 1-171966A is directed to a polyisocyanate composition comprising a cyanurate-containing polyisocyanate prepared from hydrogenated xylylene diisocyanate and a cyanurate-containing polyisocyanate prepared from hexamethylene diisocyanate at a certain ratio.

### SUMMARY OF THE INVENTION

### PROBLEM TO BE SOLVED BY THE INVENTION

Meanwhile, in the isocyanurate-forming reaction shown in the above-described Patent Document 1, in view of production efficiency, improvement in its reaction rate is demanded. Furthermore, the above-described reaction easily produces a by-product such as polyamide (nylon, etc.) and causes gelation, and therefore suppression of gelation is demanded.

An object of the present invention is to provide a method for producing a polyisocyanurate composition with which gelation can be suppressed, isocyanurate formation of xylylenediisocyanate can be achieved with excellent reaction rate, and production efficiency can be improved; and a polyisocyanurate composition produced by the method for producing a polyisocyanurate composition.

### MEANS FOR SOLVING THE PROBLEM

A method for producing a polyisocyanurate composition of the present invention includes subjecting xylylenediisocyanate to isocyanurate-forming reaction in the presence of an isocyanurate-forming catalyst, wherein the isocyanurate-forming catalyst is hydroxide of tetrabutyl ammonium.

In the method for producing a polyisocyanurate composition of the present invention, it is preferable that the reaction temperature in the isocyanurate-forming reaction is 60°C or more and 80°C or less.

In the method for producing a polyisocyanurate composition of the present invention, it is preferable that organic phosphite is blended as a promoter in the isocyanurate-forming reaction.

In the method for producing a polyisocyanurate composition of the present invention, it is preferable that the promoter is aliphatic organic phosphite.

A polyisocyanurate composition of the present invention is produced by the above-described method for producing a polyisocyanurate composition.

### Effects of the Invention

With the method for producing a polyisocyanurate composition of the present invention, gelation can be suppressed, and isocyanurate formation of xylylenediisocyanate can be achieved with excellent reaction rate, and therefore excellent production efficiency can be achieved.

Therefore, the polyisocyanurate composition of the present invention has excellent production efficiency.

### DESCRIPTION OF EMBODIMENTS

In the method for producing a polyisocyanurate composition of the present invention, xylylenediisocyanate is subjected to isocyanurate-forming reaction in the presence of an isocyanurate-forming catalyst.

Examples of xylylenediisocyanate include structural isomers of 1,2-xylylenediisocyanate (o-xylylenediisocyanate (o-XDI)), 1,3-xylylenediisocyanate (m-xylylenediisocyanate (m-XDI)), and 1,4-xylylenediisocyanate (p-xylylenediisocyanate (p-XDI)).

These xylylenediisocyanates may be used singly or in combination of two or more. For xylylenediisocyanate, preferably 1,3-xylylenediisocyanate, 1,4-xylylenediisocyanate, more preferably 1,3-xylylenediisocyanate is used.

In the present invention, for the isocyanurate-forming catalyst, tetrabutylammonium is used.

When the above-described catalyst is used for the isocyanurate-forming catalyst, gelation can be suppressed, and in particular isocyanurate formation of xylylenediisocyanate can be achieved with excellent reaction rate, and therefore excellent production efficiency can be achieved.

The isocyanurate-forming catalyst can be used as the one having a solid content of 100%, or can be diluted with an organic solvent such as alcohol (e.g., methanol, isopropylalcohol, isobutylalcohol, etc.) at a suitable ratio.

The isocyanurate-forming catalyst can be added in an amount (solid content based) relative to 100 parts by mass of xylylenediisocyanate of, for example, 0.001 parts by mass or more, preferably 0.002 parts by mass or more, more preferably 0.005 parts by mass or more, and for example, 0.5 parts by mass or less, preferably 0.2 parts by mass or less, more preferably 0.1 parts by mass or less.

Then, in this method, xylylenediisocyanate is blended with the isocyanurate-forming catalyst at the above-described mixing ratio, and the mixture is heated, thereby causing isocyanurate-forming reaction.

The reaction conditions for the isocyanurate-forming reaction are as follows: for example, under an atmosphere of inert gas such as nitrogen gas, normal pressure (atmospheric pressure), a reaction temperature (maximum temperature reached) of, for example, 20°C or more, preferably more than 40°C, more preferably 45°C or more, still more preferably 60°C or more, and for example, 90°C or less, preferably 80°C or less. The reaction time is, for example, 30 minutes or more, preferably 60 minutes or more, more preferably 120 minutes or more, and for example, 720 minutes or less, preferably 600 minutes or less, more preferably 480 minutes or less.

In the above-described reaction, to adjust the isocyanurate formation, for example, organic phosphite described in Japanese Unexamined Patent Publication No. Sho. 61-129173 can be blended as a promoter.

Examples of the organic phosphite include aliphatic organic phosphite and aromatic organic phosphite.

Examples of the aliphatic organic phosphite include alkyl monophosphites such as triethyl phosphite, tributyl phosphite, tris (2-ethylhexyl) phosphite, tridecyl phosphite, trilauryl phosphite, tris (tridecyl) phosphite, and tristearyl phosphite; di, tri, or tetra phosphites derived from aliphatic polyhydric alcohols such as distearyl·pentaerythrityl·diphosphite, di·dodecyl·pentaerythritol·diphosphite, di·tridecyl·pentaerythritol·diphosphite, and tripentaerythritol·tri phosphite; and furthermore, alicyclic poly phosphites such as a hydrogenated bisphenol A phosphite polymer (molecular weight 2400 to 3000), and tris (2,3-dichloropropyl) phosphite.

Examples of the aromatic organic phosphite include aryl monophosphites such as triphenyl phosphite, tris (nonylphenyl) phosphite, tris (2,4-di-t-butylphenyl) phosphite, diphenyldecyl phosphite, and diphenyl (tridecyl) phosphite; di, tri, or tetra phosphite derived from aromatic polyhydric alcohol such as dinonylphenyl·pentaerythritol·diphosphite, tetraphenyl·tetra·tridecyl·pentaerythrityl·tetra phosphite, and tetraphenyl·dipropylene glycol·diphosphite; and furthermore, diphosphites derived from bisphenol compounds such as di·alkyl having 1 to 20 carbon atoms·bisphenol A-diphosphite, 4,4'-butylidene-bis(3-methyl-6-t-butylphenyl-di·tridecyl) phosphite.

These organic phosphites may be used singly or in combination of two or more.

For the organic phosphites, preferably, aliphatic organic phosphite is used, more preferably, alkylmonophosphite is used, and even more preferably, tridecylphosphite is used.

By blending the above-described organic phosphites as the promoter, the reaction velocity and the reaction rate can be improved, and gelation can be suppressed.

In the above-described reaction, stabilizers including a hindered phenol antioxidant such as 2,6-di(tert-butyl)-4-methylphenol (also called: dibutylhydroxytoluene, hereinafter may be referred to as BHT.), IRGANOX 1010, IRGANOX 1076, IRGANOX 1135, and IRGANOX 245 (all manufactured by Ciba Japan K.K., trade name) can be added.

Furthermore, in the above-described reaction, as necessary, a known reaction solvent can be blended, and furthermore, a known catalyst deactivator (e.g., phosphoric acid, monochloroacetic acid, dodecylbenzenesulfonic acid, p-toluenesulfonic acid, benzoyl chloride, etc.) can be added at arbitrary timing.

Then, after completion of the reaction, unreacted xylylenediisocyanate can be removed, as necessary, by a known method.

Furthermore, in the above-described reaction, to adjust the viscosity of the polyisocyanurate composition, as necessary, alcohols can be blended.

Alcohols can be blended, for example, by the following methods: first, xylylenediisocyanate is allowed to react with alcohols, and then after subjecting the product to isocyanurate-forming reaction in the presence of an isocyanurate-forming catalyst, unreacted xylylenediisocyanate is removed; and for example, first, only xylylenediisocyanate is subjected to isocyanurate formation in the above-described method, thereafter unreacted xylylenediisocyanate is removed, and thereafter, produced polyisocyanurate is allowed to react with alcohols.

Preferably, first, xylylenediisocyanate is allowed to react with alcohols, and then after the product is subjected to isocyanurate-forming reaction in the presence of an isocyanurate-forming catalyst, unreacted xylylenediisocyanate is removed.

To be specific, in this method, first, xylylenediisocyanate is mixed with alcohols and the mixture is allowed to react.

In the present invention, examples of alcohols include monohydric alcohol, dihydric alcohol, trihydric alcohol, and an alcohol having four or more OH groups.

Examples of the monohydric alcohol include a straight chain monohydric alcohol, and a branched monohydric alcohol.

Examples of the straight chain monohydric alcohol include methanol, ethanol, n-propanol, n-butanol, n-pentanol, n-hexanol, n-heptanol, n-octanol, n-nonanol, n-decanol, n-undecanol, n-dodecanol (lauryl alcohol), n-tridecanol, n-tetradecanol, n-pentadecanol, n-hexadecanol, n-heptadecanol, n-octadecanol (stearyl alcohol), n-nonadecanol, and eicosanol.

Examples of the branched monohydric alcohol include isopropanol, isobutanol, sec-butanol, tert-butanol, isopentanol, isohexanol, isoheptanol, isooctanol, isononanol, isodecanol, 5-ethyl-2-nonanol, trimethylnonylalcohol, 2-hexyldecanol, 3,9-diethyl-6-tridecanol, 2-isoheptylisoundecanol, 2-octyldodecanol, and other branched alkanols (C (number of carbon, the same applies to the following) 5 to 20).

Examples of the dihydric alcohol include straight chain dihydric alcohols such as ethylene glycol, 1,3-propanediol, 1,4-butyleneglycol, 1,5-pentanediol, 1,6-hexanediol, 1,4-dihydroxy-2-butene, diethylene glycol, triethylene glycol, dipropylene glycol, and other straight chain alkane (C7 to 20) diol; branched dihydric alcohols such as 1,2-propanediol, 1,3-butyleneglycol (also called: 1,3-butanediol), 1,2-butyleneglycol, neopentyl glycol, 3-methyl-1,5-pentanediol, 2,2,2-trimethylpentanediol, 3,3-dimethylolheptane, 2,6-dimethyl-1-octene-3,8-diol, and other branched alkane (C7 to 20) diol; and 1,3-or 1,4-cyclohexanedimethanol and a mixture thereof, 1,3-or 1,4-cyclohexanediol and a mixture thereof, hydrogenated bisphenol A, and bisphenol A.

Examples of the trihydric alcohol include glycerin, and trimethylolpropane.

Examples of the alcohol having four or more OH groups include tetramethylolmethane, D-sorbitol, xylitol, and D-mannitol.

These alcohols contain one or more hydroxy group in its molecule, and the molecular structure other than that is not particularly limited, as long as it does not hinder excellent effects of the present invention. For example, an ester group, an ether group, a cyclohexane ring, and an aromatic ring may be contained in its molecule. Examples of the alcohols include an ether group-containing monohydric alcohol of an addition polymerization product (random and/or block polymer of two or more types of alkylene oxides) of the above-described monohydric alcohol and alkylene oxide (e.g., ethyleneoxide, propyleneoxide, etc.), and an ester group-containing monohydric alcohol of an addition polymerization product of the above-described monohydric alcohol and lactone (e.g., *ε* -caprolactone, *δ* -valerolactone, etc.).

These alcohols may be used singly or in combination of two or more.

As alcohols, preferably, mono and dihydric alcohols are used, and as the mono and dihydric alcohols, preferably mono and dihydric alcohol having 1 to 20 carbon atoms, and more preferably mono and dihydric alcohol having 1 to 15 carbon atoms, and more preferably mono and dihydric alcohol having 1 to 10 carbon atoms, and particularly preferably mono and dihydric alcohol having 2 to 6 carbon atoms are used. For the monohydric and dihydric alcohols, preferably, branched monohydric and dihydric alcohols are used, more preferably, branched dihydric alcohols are used.

Alcohols are used so that the number of the average functional group in the produced polyisocyanurate composition is 2 or more, and the mixing ratio of the alcohols relative to 100 parts by mass of the xylylenediisocyanate is, for example, 0.1 parts by mass or more, preferably 0.5 parts by mass or more, more preferably 1 part by mass or more, and for example, 40 parts by mass or less, preferably 20 parts by mass or less, more preferably 10 parts by mass or less.

In this reaction, xylylenediisocyanate is blended with alcohols at a mixing ratio such that the equivalent ratio (NCO/OH) of the isocyanate group of xylylenediisocyanate relative to the hydroxy group of alcohols is, for example, 5 or more, preferably 10 or more, more preferably 20 or more, still more preferably 25 or more, and generally 1000 or less.

Furthermore, in this reaction, in the range that does not hinder the excellent effects of the present invention, as necessary, the above-described alcohols can be used in combination with, for example, active hydrogen group-containing compounds such as thiols, oximes, lactams, phenols, and βdiketones.

The reaction conditions for the reaction of xylylenediisocyanate with alcohols are as follows: for example, under an atmosphere of inert gas such as nitrogen gas and normal pressure (atmospheric pressure), the reaction temperature of, for example, room temperature (e.g., 25°C) or more, preferably 40°C or more, and for example, 100°C or less, preferably 90°C or less. The reaction time is, for example, 0.05 hours or more, preferably 0.2 hours or more, and for example, 10 hours or less, preferably 6 hours or less.

Then, in this method, the isocyanurate-forming catalyst is blended at the above-described mixing ratio to the produced reaction liquid, and the reaction product of xylylenediisocyanate and alcohols is subjected to isocyanurate-forming reaction. The reaction conditions in the isocyanurate formation are the same as the above-described. After the completion of reaction, the unreacted xylylenediisocyanate is removed, as necessary, by a known removal method such as distillation.

The polyisocyanurate composition can be produced in this manner.

When only xylylenediisocyanate is subjected to isocyanurate formation, and then thereafter unreacted xylylenediisocyanate is removed and the produced polyisocyanate is allowed to react with alcohols (latter method in the above description), the reaction of polyisocyanate with alcohols is general urethane-forming reaction. The reaction conditions for such a urethane-forming reaction are, for example, a room temperature to 100° C, preferably 40 to 90° C.

In the above-described urethane-forming reaction, as necessary, for example, a known urethane-forming catalyst such as amines and organic metal compounds can be added.

Examples of amines include tertiary amines such as triethylamine, triethylenediamine, bis-(2-dimethylaminoethyl) ether, and N-methylmorpholine; quaternary ammonium salts such as tetraethyl hydroxyl ammonium; and imidazoles such as imidazole and 2-ethyl-4-methylimidazole.

Examples of organic metal compounds include organic tin compounds such as tin acetate, stannous octoate, stannous oleate, tin laurate, dibutyl tin diacetate, dimethyl tin dilaurate, dibutyl tin dilaurate, dibutyl tin dimercaptide, dibutyl tin maleate, dibutyl tin dilaurate, dibutyl tin dineodecanoate, dioctyl tin dimercaptide, dioctyl tin dilaurylate, and dibutyl tin dichloride; organic lead compounds such as lead octanoate and lead naphthenate; organic nickel compound such as nickel naphthenate; organic cobalt compounds such as cobalt naphthenate; organic copper compounds such as copper octenate; organic bismuth compounds such as bismuth octylate and bismuth neodecanoate.

Examples of urethane-forming catalysts also include potassium salts such as potassium carbonate, potassium acetate, and potassium octoate.

These urethane-forming catalysts may be used singly or in combination of two or more.

The polyisocyanurate composition can be produced in this manner.

Furthermore, in this method, as necessary, after the isocyanurate-forming reaction, a compound containing a sulfonamide group can be blended.

For the compound containing a sulfonamide group, for example, aromatic sulfonamides and aliphatic sulfonamides are used.

Examples of aromatic sulfonamides include benzene sulfonamide, dimethylbenzene sulfonamide, sulfanilamide, o- and p-toluene sulfonamide, hydroxynaphthalene sulfonamide, naphthalene-1-sulfonamide, naphthalene-2-sulfonamide, m-nitrobenzene sulfonamide, and p-chlorobenzene sulfonamide.

Examples of aliphatic sulfonamides include methane sulfonamide, N,N-dimethylmethane sulfonamide, N,N-dimethylethane sulfonamide, N,N-diethylmethane sulfonamide, N-methoxymethane sulfonamide, N-dodecylmethane sulfonamide, N-cyclohexyl-1-butanesulfonamide, and 2-aminoethane sulfonamide.

These compounds containing a sulfonamide group may be used singly or in combination of two or more.

As the compound containing a sulfonamide group, preferably, aromatic sulfonamides are used, more preferably, o- or p-toluene sulfonamides are used.

The compound containing a sulfonamide group is blended, for example, after the isocyanurate-forming reaction of xylylenediisocyanate is completed, to the reaction liquid. The compound containing a sulfonamide group can also be blended, as necessary, after unreacted xylylenediisocyanate (monomer) is removed from the reaction liquid, to the reaction liquid.

The mixing ratio of the compound containing a sulfonamide group relative to 100 parts by mass of the xylylenediisocyanate is, 0.001 to 0.5 parts by mass, preferably 0.005 to 0.4 parts by mass, more preferably 0.01 to 0.3 parts by mass. That is, the compound containing a sulfonamide group relative to a total amount of xylylenediisocyanate is, for example, 10 to 5000 ppm, preferably 50 to 4000 ppm, more preferably 100 to 3000 ppm.

When the compound containing a sulfonamide group is added at the above-described ratio, improvement in storage stability of the polyisocyanurate composition can be achieved.

The thus produced polyisocyanurate composition has an isocyanate group concentration of, for example, 15 mass% or more, preferably 16 mass% or more, and for example, 22 mass% or less, preferably 21 mass% or less.

The thus produced polyisocyanurate composition has an isocyanate monomer concentration (unreacted xylylenediisocyanate concentration) of, for example, 5 mass% or less, preferably 2 mass% or less, more preferably 1 mass% or less.

The polyisocyanurate composition can be diluted, as necessary, with an organic solvent.

Examples of organic solvents include ketones such as acetone, methyl ethyl ketone, methylisobutylketone, and cyclohexanone; nitriles such as acetonitrile; alkyl esters such as methyl acetate, ethyl acetate, butyl acetate, and isobutyl acetate; aliphatic hydrocarbons such as n-hexane, n-heptane, and octane; alicyclic hydrocarbons such as cyclohexane and methylcyclohexane; aromatic hydrocarbons such as toluene, xylene, and ethylbenzene; glycol ether esters such as methyl cellosolve acetate, ethyl cellosolve acetate, methyl carbitol acetate, ethyl carbitol acetate, ethylene glycol ethylether acetate, propylene glycol methylether acetate, 3-methyl-3-methoxybutyl acetate, and ethyl-3-ethoxypropionate; ethers such as diethylether, tetrahydrofuran, and dioxane; halogenated aliphatic hydrocarbons such as methyl chloride, methylene chloride, chloroform, carbon tetrachloride, methyl bromide, methylene iodide, and dichloroethane; polar aprotic solvents such as N-methyl pyrrolidone, dimethylformamide, N,N'-dimethylacetamide, dimethyl sulfoxide, and hexamethyl phosphoramide.

Examples of organic solvents also include nonpolar solvents (nonpolar organic solvents), and examples of nonpolar solvents include those nonpolar organic solvents having an aniline point of, for example, 10 to 70° C, preferably 12 to 65° C and having low toxicity and solvency, such as aliphatic, naphthene hydrocarbon organic solvent; and vegetable oils typically represented by turpentine oil.

The nonpolar organic solvents can be obtained from commercially available products, and examples of those commercially available products include petroleum hydrocarbon organic solvents such as Haws (manufactured by Shell Chemicals, aniline point 15° C), Swasol 310 (manufactured by Maruzen Petrochemical, aniline point 16° C), Esso Naphtha No. 6 (manufactured by Exxon Mobil Chemical, aniline point 43° C), Laws (manufactured by Shell Chemicals, aniline point 43° C), Esso Naphtha No. 5 (manufactured by Exxon Mobil Corporation, aniline point 55° C), and pegasol 3040 (manufactured by Exxon Mobil Corporation, aniline point 55° C); and also methylcyclohexane (aniline point 40° C), ethylcyclohexane (aniline point 44° C), and turpentine oils such as gum turpentine N (manufactured by YASUHARA CHEMICAL CO., LTD., aniline point 27° C).

The polyisocyanurate composition can be mixed with these organic solvents at any rate.

When the polyisocyanurate composition is diluted with the organic solvent, the polyisocyanurate composition concentration is, for example, 20 mass% or more, preferably 30 mass% or more, and for example, 95 mass% or less, preferably 90 mass% or less.

In this case, the viscosity at 25°C is adjusted to be, for example, 10 mPa·s or more, preferably 20 mPa·s or more, and for example, 10000 mPa·s or less, preferably 5000 mPa·s or less.

With such a method for producing a polyisocyanurate composition, gelation can be suppressed, and the isocyanurate formation of xylylenediisocyanate can be achieved with excellent reaction rate, and therefore excellent production efficiency can be achieved. Therefore, the produced polyisocyanurate composition is excellent in production efficiency.

Therefore, the polyisocyanurate composition can be suitably used in production of polyurethane resin.

Polyurethane resin can be produced by allowing the above-described polyisocyanurate composition to react with an active hydrogen group-containing compound.

In the present invention, examples of the active hydrogen group-containing compound include a polyol component and a polyamine component, and preferably, a polyol component is used.

Examples of polyol component in the present invention include low-molecular-weight polyols and high-molecular weight polyols.

The low molecular-weight polyol is a compound having two or more hydroxyl groups and a number average molecular weight of less than 300, preferably less than 400, and examples thereof include dihydric alcohols such as ethylene glycol, propylene glycol, 1,3-propanediol, 1,4-butyleneglycol, 1,3-butyleneglycol, 1,2-butyleneglycol, 1,5-pentanediol, 1,6-hexanediol, neopentyl glycol, 3-methyl-1,5-pentanediol, 2,2,2-trimethylpentanediol, 3,3-dimethylolheptane, alkane (C 7 to 20) diol, 1,3-or 1,4-cyclohexanedimethanol and a mixture thereof, 1,3-or 1,4-cyclohexanediol and a mixture thereof, hydrogenated bisphenol A, 1,4-dihydroxy-2-butene, 2,6-dimethyl-1-octene-3,8-diol, bisphenol A, diethylene glycol, triethylene glycol, and dipropylene glycol; trihydric alcohols such as glycerin, trimethylolpropane, and triisopropanolamine; tetrahydric alcohols such as tetramethylolmethane (pentaerythritol) and diglycerol; pentahydric alcohols such as xylitol; hexahydric alcohols such as sorbitol, mannitol, allitol, iditol, dulcitol, altritol, inositol, and dipentaerythritol; heptahydric alcohols such as perseitol; and octahydric alcohols such as sucrose.

These low-molecular-weight polyols may be used singly or in combination of two or more.

The high molecular weight polyol is a compound having two or more hydroxyl groups and a number average molecular weight of 300 or more, preferably 400 or more, still more preferably 500 or more, and examples thereof include polyetherpolyol (e.g., polyoxyalkylenepolyol, polytetramethylene ether polyol, etc.), polyesterpolyol (e.g., adipic acid polyesterpolyol, phthalic acid polyesterpolyol, lactone polyesterpolyol, etc.), polycarbonatepolyol, polyurethane polyol (e.g., polyol produced by subjecting, for example, polyetherpolyol, polyesterpolyol, or polycarbonatepolyol to urethane modification with polyisocyanate), epoxy polyol, vegetable oil polyol, polyolefinpolyol, acrylic polyol, and vinyl monomer-modified polyol.

These high molecular weight polyols may be used singly or in combination of two or more.

For the high molecular weight polyol, preferably, polyetherpolyol, polyesterpolyol, and polycarbonatepolyol are used.

The polyurethane resin can be produced by, for example, polymerization methods such as bulk polymerization and solution polymerization.

In bulk polymerization, for example, the active hydrogen group-containing compound is added to the polyisocyanurate composition while stirring under nitrogen flow, and reaction is allowed to occur at a reaction temperature of 50 to 250°C, even more preferably 50 to 200°C, for about 0.5 to 15 hours.

In the solution polymerization, the polyisocyanurate composition and the active hydrogen group-containing compound are added to an organic solvent of those organic solvents used for the above-described dilution of the polyisocyanurate composition, and the mixture is allowed to react at a reaction temperature of 50 to 120°C, preferably 50 to 100°C, for about 0.5 to 15 hours.

Furthermore, in the above-described polymerization reaction, as necessary, for example, the above-described urethane-forming catalyst can be added.

Furthermore, in bulk polymerization and solution polymerization, for example, the polyisocyanurate composition and the active hydrogen group-containing compound are blended so that the equivalent ratio (NCO/active hydrogen group) of the isocyanate group in the polyisocyanurate composition relative to the active hydrogen group (hydroxyl group, mercapto group, amino group) in the active hydrogen group-containing compound is, for example, 0.75 to 1.3, preferably 0.9 to 1.1.

Further, when the above polymerization reaction is more industrially carried out, the polyurethane resin can be obtained by a known process such as one shot process and prepolymer process.

In the one shot process, for example, the polyisocyanurate composition and the active hydrogen group-containing compound are formulated (mixed) so that the equivalent ratio (NCO/active hydrogen group) of the isocyanate group in the polyisocyanurate composition relative to the active hydrogen group in the active hydrogen group (hydroxyl group, mercapto group, amino group) -containing compound is, for example, 0.75 to 1.3, preferably 0.9 to 1.1, and thereafter, for example, subjected to curing reaction at, for example, room temperature to 250°C, preferably, room temperature to 200°C, and for example, 5 minutes to 72 hours, preferably 4 to 24 hours. The curing temperature may be a constant temperature, or may be increased/decreased stepwise.

In the prepolymer process, for example, first, the polyisocyanurate composition is allowed to react with a portion of the active hydrogen group-containing compound (preferably, high molecular weight polyol) to synthesize an isocyanate group-terminated prepolymer having an isocyanate group at the terminal of the molecule. Then, the produced isocyanate group-terminated prepolymer is allowed to react with the remaining portion of the active hydrogen group-containing compound (preferably, low molecular-weight polyol and/or polyamine component) to cause curing reaction. In the prepolymer process, the remaining portion of the active hydrogen group-containing compound is used as the chain extender.

To synthesize the isocyanate group-terminated prepolymer, the polyisocyanurate composition is formulated (mixed) with a portion of the active hydrogen group-containing compound so that the equivalent ratio (NCO/active hydrogen group) of the isocyanate group in the polyisocyanurate composition relative to the active hydrogen group in the portion of the active hydrogen group-containing compound is, for example, 1.1 to 20, preferably 1.3 to 10, still more preferably 1.3 to 6, and the mixture is allowed to react in a reaction vessel at, for example, room temperature to 150°C, preferably 50 to 120°C, for, for example, 0.5 to 18 hours, preferably 2 to 10 hours. In this reaction, as necessary, the above-described urethane-forming catalyst may be added, and after the completion of the reaction, as necessary, the unreacted polyisocyanurate composition can be removed, for example, by a known removal method such as distillation and extraction.

Then, to allow the produced isocyanate group-terminated prepolymer to react with the remaining portion of the active hydrogen group-containing compound, the isocyanate group-terminated prepolymer is formulated (mixed) with the remaining portion of the active hydrogen group-containing compound so that the equivalent ratio (NCO/active hydrogen group) of the isocyanate group in the isocyanate group-terminated prepolymer relative to the active hydrogen group in the remaining portion of the active hydrogen group-containing compound is, for example, 0.75 to 1.3, preferably 0.9 to 1.1, and curing reaction is allowed to occur at, for example, room temperature to 250°C, preferably, room temperature to 200°C for, for example, 5 minutes to 72 hours, preferably 1 to 24 hours.

The polyurethane resin can be produced in this manner.

When the polyurethane resin is produced, as necessary, known additives, for example, a plasticizer, an anti-blocking agent, a heat-resistant stabilizer, a light stabilizer, an antioxidant, a mold release agent, a catalyst, and furthermore, a pigment, a dye, a lubricant, a filler, and a hydrolysis prevention agent can be further added at a suitable ratio. These additives may be added at the time of synthesizing components, or may be added at the time of mixing and dissolving components, or may be added after the synthesis.

Such a polyurethane resin is produced by using the polyisocyanurate composition of the present invention, and therefore excellent production efficiency can be achieved.

Therefore, such a polyurethane resin can be widely used in various fields such as, for example, plastic paint, automotive paint, a film coating agent, various inks, various adhesives, pressure-sensitive adhesives, sealing material, various microcapsules, plastic lens, artificial and synthetic leather, RIM product, slush powder, elastic molded articles (spandex), and urethane foam.

### Examples

In the following, the present invention will be described in more detail with reference to Examples and Comparative Examples, but the present invention is not limited thereto. In the description below, "parts" and "%" are mass-based unless otherwise specified. The numeral values shown in Examples below can be replaced with the numeral values (that is, upper limit value or lower limit value) used in embodiments.

The measurement methods used in Examples and Comparative Examples are described below.

### <Reaction rate (%)·reaction velocity>

The reaction rate in the isocyanurate-forming reaction was determined by measuring the isocyanate group concentration (method in conformity with JIS K-1556), and obtaining the reduction rate.

Furthermore, the reaction rate was plotted against time, thereby evaluating the reaction velocity.

The reaction velocity was evaluated as fast (EXCELLENT) when the reaction rate reached 18% or more within 300 minutes, and evaluated as slightly slow (BAD) when the reaction rate did not reach 18% or more within 300 minutes.

### <Gelation>

Presence or absence of gelation was evaluated by visually observing the reaction liquid after the isocyanurate-forming reaction.

Those reaction liquids that were transparent, had flowability, and without insoluble matter were evaluated as EXCELLENT, and those with insoluble matter were evaluated as BAD. Of those reaction liquids with no insoluble matter after the isocyanurate-forming reaction, those reaction liquids which produced cloudy white matter next day were evaluated as GOOD.

### Example 1 (Reference Example)

In a reactor equipped with a thermometer, a stirrer, a nitrogen inlet tube, and a condenser tube, 100 parts by mass of m-xylylenediisocyanate (manufactured by Mitsui Chemicals, Inc., XDI), 0.02 parts by mass of 2,6-di (tert-butyl)-4-methylphenol (also called: dibutylhydroxytoluene, BHT, hindered phenol antioxidant), and 0.05 parts by mass of tetraphenyl·dipropylene glycol·diphosphite (trade name JPP-100, manufactured by Johoku Chemical Co. Ltd., organic phosphites, promoter) were mixed in a nitrogen atmosphere, and thereafter, to the mixture liquid, 0.02 parts by mass of hydroxide of trimethylbenzylammonium (40%methanol solution) was blended as an isocyanurate-forming catalyst, and they were allowed to react at a reaction start temperature of 50°C for 200 minutes. The maximum temperature reached during the reaction was 57°C.

The obtained reaction mixture liquid was passed through a thin film distillation device (temperature 150°C, degree of vacuum 93.3 Pa) to remove unreacted xylylenediisocyanate, thereby producing a polyisocyanurate composition. The reaction rate in this reaction was 21.5%, the reaction velocity was fast, and no gelation was found in the reaction liquid.

### Example 2

A polyisocyanurate composition was produced in the same manner as in Example 1, except that 0.02 parts by mass of hydroxide of tetrabutylammonium (37% methanol solution) was blended instead of hydroxide of trimethylbenzylammonium (40% methanol solution) as an isocyanurate-forming catalyst, and the reaction time was changed to 300 minutes. The maximum temperature reached during the reaction was 56°C. The reaction rate in this reaction was 21.7%, the reaction velocity was fast, and no gelation was found in the reaction liquid.

### Example 3 (Reference Example)

In a reactor equipped with a thermometer, a stirrer, a nitrogen inlet tube, and a condenser tube, 100 parts by mass of m-xylylenediisocyanate (manufactured by Mitsui Chemicals, Inc., XDI), 0.02 parts by mass of 2,6-di (tert-butyl)-4-methylphenol (also called: dibutylhydroxytoluene, BHT, hindered phenol antioxidant), and 0.05 parts by mass of tetraphenyl·dipropylene glycol·diphosphite (organic phosphites, promoter) in a nitrogen atmosphere, and thereafter 2 parts by mass of 1,3-butanediol was added to the mixture, and the temperature of the mixture liquid was increased to 70°C.

Then, after reaction at the same temperature for 180 minutes, the temperature was decreased to 50°C. Then, 0.02 parts by mass of hydroxide of trimethylbenzylammonium (40% methanol solution) was added as an isocyanurate-forming catalyst, and after reaction was continued for 280 minutes at the reaction start temperature of 50°C, the reaction was terminated. The maximum temperature reached during the reaction was 61°C.

The obtained reaction mixture liquid was passed through a thin film distillation device (temperature 150°C, degree of vacuum 50 Pa) to remove unreacted xylylenediisocyanate, thereby producing a polyisocyanurate composition. The reaction rate in this reaction was 18.0%, the reaction velocity was fast, and no gelation was found in the reaction liquid.

### Example 4

A polyisocyanurate composition was produced in the same manner as in Example 2, except that the reaction start temperature was changed to 60°C. The maximum temperature reached during the reaction was 74°C. The reaction rate in this reaction was 25.8%, the reaction velocity was fast, and no gelation was found in the reaction liquid.

### Example 5

A polyisocyanurate composition was produced in the same manner as in Example 2, except that 0.04 parts by mass of hydroxide of tetrabutylammonium (37% methanol solution) was blended, the reaction start temperature was changed to 70°C, and the reaction time was changed to 320 minutes. The maximum temperature reached during the reaction was 79°C. The reaction rate in this reaction was 33.5%, the reaction velocity was fast, and no gelation was found in the reaction liquid.

### Example 6

A polyisocyanurate composition was produced in the same manner as in Example 4, except that 0.05 parts by mass of tridecylphosphite (trade name JP-310, manufactured by Johoku Chemical Co. Ltd., organic phosphites, promoter) was blended instead of 0.05 parts by mass of tetraphenyl·dipropylene glycol·diphosphite (trade nameJPP-100, manufactured by Johoku Chemical Co. Ltd., organic phosphites, promoter). The maximum temperature reached during the reaction was 76°C. The reaction rate in this reaction was 30.0%, the reaction velocity was fast, and no gelation was found in the reaction liquid.

### Example 7

A polyisocyanurate composition was produced in the same manner as in Example 4, except that tetraphenyl · dipropylene glycol · diphosphite (trade name JPP-100, manufactured by Johoku Chemical Co. Ltd., organic phosphites, promoter) was not blended. The maximum temperature reached during the reaction was 75°C. The reaction rate in this reaction was 25.1%, the reaction velocity was fast, and no gelation was found in the reaction liquid.

### Example 8

A polyisocyanurate composition was produced in the same manner as in Example 2, except that the reaction start temperature was changed to 80°C, and the reaction time was changed to 200 minutes. The maximum temperature reached during the reaction was 90°C. The reaction rate in this reaction was 35.1%, the reaction velocity was fast, and no gelation was found in the reaction liquid, but white cloudy matter was found the next day.

### Comparative Example 1

A polyisocyanurate composition was produced in the same manner as in Example 1, except that 0.05 parts by mass as a solid content of TAP (manufactured by Kayaku Akuzo Corporation, 2,4,6-tris (dimethylaminomethyl) phenol) was blended as an isocyanurate-forming catalyst instead of hydroxide of trimethylbenzylammonium (40% methanol solution), and the reaction time was changed to 300 minutes. The maximum temperature reached during the reaction was 52°C. The reaction rate in this reaction was 16.9%, the reaction velocity was slightly slow, and no gelation was found in the reaction liquid.

### Comparative Example 2

A polyisocyanurate composition was produced in the same manner as in Example 1, except that 0.02 parts by mass as a solid content of DABCO-TMR (N-(2-hydroxypropyl)-N,N,N-trimethylammonium-2-ethylhexanoate, manufactured by Air Products and Chemicals, Inc.) was blended as an isocyanurate-forming catalyst instead of hydroxide of trimethylbenzylammonium (40% methanol solution), and the reaction time was changed to 300 minutes. The maximum temperature reached during the reaction was 55°C. The reaction rate in this reaction was 19.7%, the reaction velocity was slightly slow, and slight gelation was found in the reaction liquid.

### Comparative Example 3

Reaction was performed in the same manner as in Example 1, except that 0.2 parts by mass as a solid content of magnesium salt of naphthenic acid (magnesium content 2%, 59% mineral spirit solution) was blended as an isocyanurate-forming catalyst instead of hydroxide of trimethylbenzylammonium (40% methanol solution), and the reaction start temperature was changed to 70°C; however, sufficient amount of polyisocyanurate composition could not be obtained even after passing 7 hours. The maximum temperature reached during the reaction was 73°C. The reaction rate in this reaction was 1.1%.

**[Table 1]**

| No. | | Example 1^{∗} | Example 2 | Example 3^{∗} | Example 4 |
|---|---|---|---|---|---|
| Mixing formulation (parts by mass) | XDI | 100 | 100 | 100 | 100 |
| | 1,3-BG | - | - | 2 | - |
| | TMB | 0.02 | - | 0.02 | - |
| | TBAOH | - | 0.02 | - | 0.02 |
| | JPP-100 | 0.05 | 0.05 | 0.05 | 0.05 |
| | JP-310 | - | - | - | - |
| Reaction conditions | Reaction start temperature | 50°C | 50°C | 50°C | 60°C |
| | Highest temperature reached | 57°C | 56°C | 61°C | 74°C |
| | Reaction time | 200min | 300min | 280min | 300min |
| Evaluation | Reaction rate | 21.5% | 21.7% | 18.0% | 25.8% |
| | Reaction velocity | EXCELLENT | EXCELLENT | EXCELLENT | EXCELLENT |
| | Gelation | EXCELLENT | EXCELLENT | EXCELLENT | EXCELLENT |

| No. | | Example 5 | Example 6 | Example 7 | Example 8 |
|---|---|---|---|---|---|
| Mixing formulation (parts by mass) | XDI | 100 | 100 | 100 | 100 |
| | 1,3-BG | - | - | - | - |
| | TMB | - | - | - | - |
| | TBAOH | 0.04 | 0.02 | 0.02 | 0.02 |
| | JPP-100 | 0.05 | - | - | 0.05 |
| | JP-310 | - | 0.05 | - | - |
| Reaction conditions | Reaction start temperature | 70°C | 60°C | 60°C | 80°C |
| | Highest temperature reached | 79°C | 76°C | 75°C | 90°C |
| | Reaction time | 320min | 300min | 300min | 200min |
| Evaluation | Reaction rate | 33.5% | 30.0% | 25.1% | 35.1% |
| | Reaction velocity | EXCELLENT | EXCELLENT | EXCELLENT | EXCELLENT |
| | Gelation | EXCELLENT | EXCELLENT | EXCELLENT | GOOD |

| No. | | Comp.Ex. 1 | Comp.Ex. 2 | Comp.Ex. 3 | |
|---|---|---|---|---|---|
| Mixing formulation (parts by mass) | XDI | 100 | 100 | 100 | |
| | TAP | 0.05 | - | - | |
| | 2%Naph.Mg | - | - | 0.2 | |
| | DABCO-TMR | - | 0.02 | - | |
| | TPP | 0.05 | 0.05 | 0.05 | |
| Reaction conditions | Reaction start temperature | 50°C | 50°C | 70°C | |
| | Highest temperature reached | 52°C | 55°C | 73°C | |
| | Reaction time | 300min | 300min | 7hr | |
| Evaluation | Reaction rate | 16.9% | 19.7% | 1.1% | |
| | Reaction velocity | BAD | EXCELLENT | | |
| | Gelation | EXCELLENT | BAD | | |

### ^{∗}Reference Examples

Details of abbreviation in Table are shown below.
XDI: m-xylylenediisocyanate (manufactured by Mitsui Chemicals, Inc.) 1,3-BG: 1,3-butanediol
TMB: hydroxide of trimethylbenzylammonium (40% methanol solution)
TBAOH: hydroxide of tetrabutylammonium (37% methanol solution)
TAP: 2,4,6-tris (dimethylaminomethyl) phenol, manufactured by Kayaku Akuzo Corporation Naph.Mg: magnesium salt of naphthenic acid
DABCO-TMR: N-(2-hydroxypropyl)-N,N,N-trimethylammonium-2-ethylhexanoate, manufactured by Air Products and Chemicals, Inc.
JPP-100: tetraphenyl·dipropylene glycol·diphosphite, manufactured by Johoku Chemical Co. Ltd., organic phosphites, promoter
JP-310: tridecylphosphite, manufactured by Johoku Chemical Co. Ltd., organic phosphites, promoter

As shown in Examples, when hydroxide of tetraalkylammonium is used as an isocyanurate-forming catalyst, gelation can be suppressed, and isocyanurate formation of xylylenediisocyanate can be achieved with excellent reaction rate.

Meanwhile, as shown in Comparative Examples, when other isocyanurate-forming catalysts are used instead of hydroxide of tetraalkylammonium, even if a larger amount than in Examples is added and the reaction time was longer than that in Examples, the reaction rate was poorer and disadvantages such as gelation occurred.

### Industrial Applicability

The method for producing a polyisocyanurate composition of the present invention, and the polyisocyanurate composition produced by the method for producing a polyisocyanurate composition are used suitably in production of polyurethane resin in various fields, such as plastic paint, automotive paint, a film coating agent, various inks, various adhesives, pressure-sensitive adhesives, sealing materials, various microcapsules, plastic lens, artificial and synthetic leather, RIM product, slush powder, elastic molded articles (spandex), and urethane foam.

## Claims

1. A method for producing a polyisocyanurate composition, the method comprising subjecting xylylenediisocyanate to isocyanurate-forming reaction in the presence of an isocyanurate-forming catalyst,
wherein the isocyanurate-forming catalyst is hydroxide of tetrabutylammonium.

2. The method for producing a polyisocyanurate composition according to Claim 1, wherein the reaction temperature in the isocyanurate-forming reaction is 60°C or more and 80°C or less.

3. The method for producing a polyisocyanurate composition according to Claim 1, wherein the organic phosphites is blended as a promoter in the isocyanurate-forming reaction.

4. The method for producing a polyisocyanurate composition according to Claim 3, wherein the promoter is aliphatic organic phosphite.

5. The method for producing a polyisocyanurate composition according to Claim 1, wherein 0.001 parts by mass or more and 0.5 parts by mass or less of the isocyanurate-forming catalyst is added relative to 100 parts by mass of xylylenediisocyanate.

6. A polyisocyanurate composition produced by the method for producing a polyisocyanurate composition according to Claim 1.

## Patentansprüche

1. Verfahren zur Herstellung einer Polyisocyanuratzusammensetzung, wobei das Verfahren eine Isocyanurat bildenden Reaktion von Xylylendiisocyanat in Gegenwart eines Isocyanurat bildenden Katalysators umfasst,
wobei der Isocyanurat bildende Katalysator Tetrabutylammoniumhydroxid ist.

2. Verfahren zur Herstellung einer Polyisocyanuratzusammensetzung gemäß Anspruch 1, wobei die Reaktionstemperatur in der Isocyanurat bildenden Reaktion 60°C oder mehr und 80°C oder weniger beträgt.

3. Verfahren zur Herstellung einer Polyisocyanuratzusammensetzung gemäß Anspruch 1, wobei die organischen Phosphite als Promotor in der Isocyanurat bildenden Reaktion beigemischt werden.

4. Verfahren zur Herstellung einer Polyisocyanuratzusammensetzung gemäß Anspruch 3, wobei der Promotor ein aliphatisches organisches Phosphit ist.

5. Verfahren zur Herstellung einer Polyisocyanuratzusammensetzung gemäß Anspruch 1, wobei 0,001 Massenteile oder mehr und 0,5 Massenteile oder weniger des Isocyanurat bildenden Katalysators, bezogen auf 100 Massenteile Xylylendiisocyanat, zugesetzt werden.

6. Polyisocyanuratzusammensetzung, hergestellt nach dem Verfahren zur Herstellung einer Polyisocyanuratzusammensetzung gemäß Anspruch 1.

## Revendications

1. Procédé de production d'une composition de polyisocyanurate, le procédé comprenant la soumission d'un xylylènediisocyanate à une réaction de formation d'isocyanurate en présence d'un catalyseur de formation d'isocyanurate,
dans lequel le catalyseur de formation d'isocyanurate est l'hydroxyde de tétrabutylammonium.

2. Procédé de production d'une composition de polyisocyanurate selon la revendication 1, dans lequel la température réactionnelle dans la réaction de formation d'isocyanurate est de 60 °C ou plus et de 80 °C ou moins.

3. Procédé de production d'une composition de polyisocyanurate selon la revendication 1, dans lequel les phosphites organiques sont mélangés en tant que promoteur dans la réaction de formation d'isocyanurate.

4. Procédé de production d'une composition de polyisocyanurate selon la revendication 3, dans lequel le promoteur est un phosphite organique aliphatique.

5. Procédé de production d'une composition de polyisocyanurate selon la revendication 1, dans lequel 0,001 partie en masse ou plus et 0,5 partie en masse ou moins du catalyseur de formation d'isocyanurate est ajoutée par rapport à 100 parties en masse de xylylènediisocyanate.

6. Composition de polyisocyanurate produite par le procédé de production d'une composition de polyisocyanurate selon la revendication 1.
